# EUROPEAN PATENT SPECIFICATION

(11) **EP 3 224 785 B1**
(45) Date of publication and mention of the grant of the patent: **08.05.2019**
(21) Application number: 15863042.6
(22) Date of filing: 18.11.2015
(51) Int. Cl.: G06Q 20/40, G06Q 50/06, G06F 21/32, H04L 29/08, A61B 5/103, A61B 5/00, G06F 1/16, H04W 12/06, H04W 84/18

(54) **ENERGY HARVESTING WEARABLE AUTHENTICATION**
AUTHENTIFIZIERUNG EINES ENERGIEGEWINNENDEN WEARABLES
AUTHENTIFICATION DE RÉCOLTE D'ÉNERGIE À L'AIDE D'UN DISPOSITIF VESTIMENTAIRE

(30) Priority: 26.11.2014 US 201414554857
(43) Date of publication of application: 04.10.2017
(73) Proprietor: Intel Corporation, Santa Clara, CA 95054 (US)
(72) Inventor: DURHAM, Lenitra M., Beaverton, Oregon 97007 (US); ANDERSON, Glen J., Beaverton, Oregon 97006 (US)
(74) Representative: Maiwald Patent- und Rechtsanwaltsgesellschaft mbH
(86) International application number: PCT/US2015/061377
(87) International publication number: WO 2016/085730

(56) References cited:
- WO-A1-2014/100045
- US-A1- 2004 177 531
- US-A1- 2006 293 606
- US-A1- 2007 247 306
- US-A1- 2007 260 421
- US-A1- 2014 089 673
- US-A1- 2014 299 783
- CAVALLARI RICCARDO ET AL: "A Survey on Wireless Body Area Networks: Technologies and Design Challenges", IEEE COMMUNICATIONS SURVEYS & TUTORIALS, IEEE, USA, vol. 16, no. 3, 1 July 2014 (2014-07-01), pages 1635-1657, XP011557059, DOI: 10.1109/SURV.2014.012214.00007 [retrieved on 2014-08-19]

## Description

### CROSS-REFERENCE TO RELATED APPLICATIONS

The present application claims the benefit of priority to U.S. Non-Provisional Patent Application No. 14/554,857 filed on November 26, 2014.

### TECHNICAL FIELD

Embodiments generally relate to user authentication. More particularly, embodiments relate to energy harvesting wearable authentication.

### BACKGROUND

Authentication may be used to grant or deny user access to various systems such as, for example, electronic commerce (e-commerce) systems, consumer devices, online accounts, and so forth. While traditional authentication approaches may have involved user entry of login and/or PIN (personal identification) information, more recent solutions may use accelerometers coupled to the leg or foot of an individual to capture gait information, wherein authentication of the individual may be performed based on the captured gait information. Such an approach may involve the installation of costly accelerometers that draw a significant amount of power during operation. Additionally, accelerometer-based authentication may be subject to gait mimicking by unauthorized individuals.

### BRIEF DESCRIPTION OF THE DRAWINGS

The various advantages of the embodiments will become apparent to one skilled in the art by reading the following specification and appended claims, and by referencing the following drawings, in which:
FIG. 1 is an illustration of an example of an energy harvesting wearable environment according to an embodiment;
FIG. 2 is a side view and a bottom view of an example of a footwear form factor according to an embodiment;
FIG. 3A is a flowchart of an example of a method of authenticating users according to an embodiment;
FIG. 3B is a flowchart of an example of a method of determining a first energy generation pattern according to an embodiment;
FIG. 4A is a block diagram of an example of a user-based system including an authentication apparatus according to an embodiment;
FIG. 4B is a block diagram of an example of a profiler according to an embodiment;
FIG. 5 is a block diagram of an example of a processor according to an embodiment; and
FIG. 6 is a block diagram of an example of a computing system according to an embodiment.

### DESCRIPTION OF EMBODIMENTS

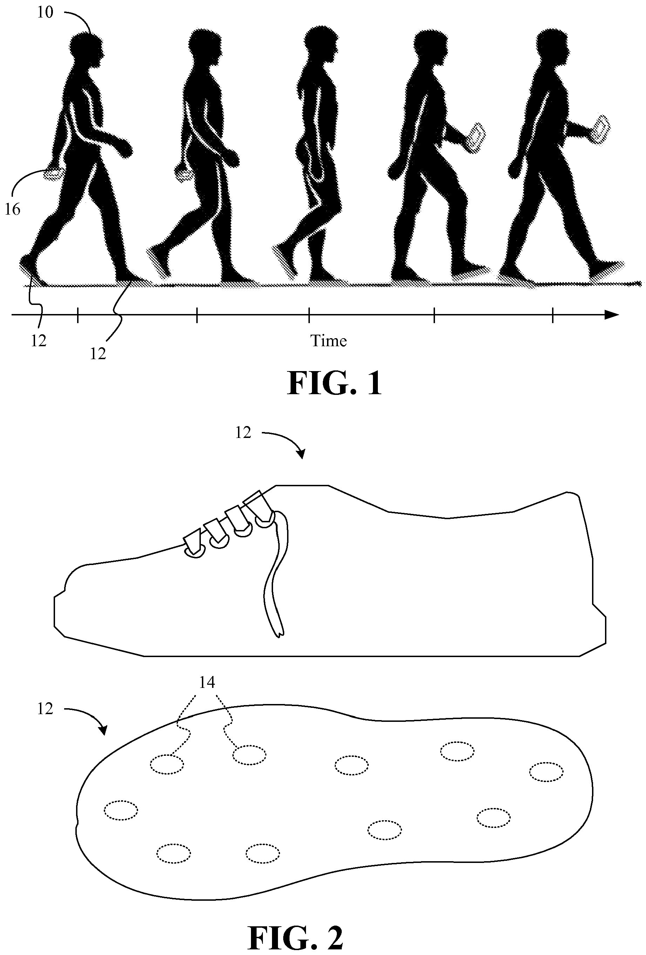

Turning now to FIG. 1, an example of an energy harvesting environment is shown in which an individual 10 has a wearable device 12 that harvests energy (e.g., electrical current, power). The harvested energy may be automatically generated when the individual 10 engages in an activity such as, for example, walking. Energy generation information collected from the wearable device 12 may be used during an authentication session to grant or deny access to an area (e.g., health club), an application accessible via a system such as, for example, a handheld device 16, or other service. The handheld device 16 may also be used to initiate the authentication session (e.g., via a gesture input, text entry, menu selection or other user input). In the illustrated example, the wearable device 12 includes a footwear form factor (e.g., shoe, boot, sandal, etc.) and may be worn on the left and/or right foot of the individual 10, although other wearable form factors may also be used depending on the circumstances. For example, a wristwear form factor might enable energy harvesting and authentication based on characteristics such as the amount of arm movement, skin temperature, lighting detected, etc., while walking or performing some other activity. Indeed, data from multiple different wearable devices may be combined during profile generation and/or user authentication. The invention is disclosed in the appended claims.

FIG. 2 demonstrates that the wearable device 12 may include one or more piezoelectric sensors 14 that generate power in response to physical deformations of the piezoelectric sensors 14 during the activity. As will be discussed in greater detail, leveraging the harvested energy from the wearable device 12 may provide a power efficient solution that eliminates any need for costly accelerometers and is relatively impervious to gait mimicking by unauthorized individuals.

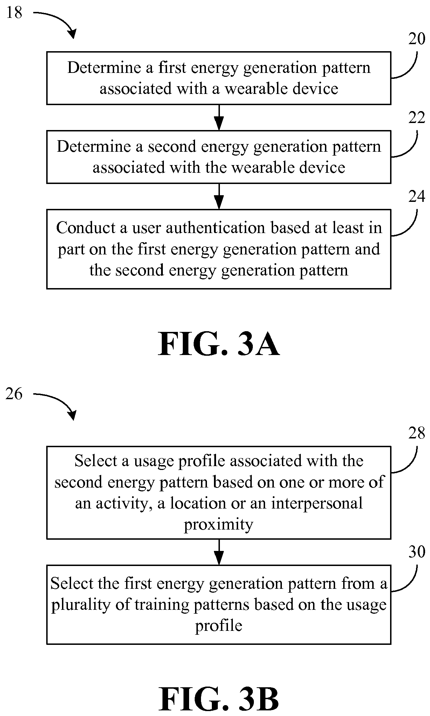

FIG. 3A shows a method 18 of authenticating users. The method 18 may be implemented as a module or related component in a set of logic instructions stored in a machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in method 18 may be written in any combination of one or more programming languages, including an object oriented programming language such as JAVA, SMALLTALK, C++ or the like and conventional procedural programming languages, such as the "C" programming language or similar programming languages.

Illustrated processing block 20 provides for determining a first energy generation pattern associated with a wearable device such as, for example, the wearable device 12 (FIGs. 1 and 2). The wearable device may therefore include one or more piezoelectric sensors that generate electrical current and/or a detectable electrical field in response to physical deformations of the sensors due to an activity (e.g., casual walking, power walking, jogging, running, cycling, etc.) conducted by an individual to which the wearable device is coupled, although other energy generation components may also be used depending on the circumstances, such as electromagnetic energy harvesting devices (e.g., rotational generators). The first energy generation pattern may generally be determined based on one or more training sessions conducted by the individual. The training sessions may be repeated in different environmental contexts as well as over time to account for physiological and/or biological changes such as, for example, weight loss, injury, aging, etc. Training sessions may also be conducted for different individuals to the extent that the wearable device is shared by multiple users.

A second energy generation pattern may be determined at block 22, wherein determining the second energy generation pattern might involve monitoring the wearable device during an authentication session initiated by, for example, a gesture input, text entry and/or menu selection. Illustrated block 24 conducts a user authentication based at least in part on the first energy generation pattern and the second energy generation pattern. Block 24 may include comparing the first energy generation pattern to the second energy generation pattern with respect to, for example, a weight, a gait rhythm, a step characteristic (e.g., level of pronation of the foot), and so forth. The compared energy generation signals may be averaged across multiple piezoelectric sensors and/or the comparison may be on a sensor-by-sensor basis.

FIG. 3B shows one example of a method 26 of determining the first energy generation pattern. The method 26 may therefore be optionally substituted for the block 20 (FIG. 3A), already discussed. The method 26 may therefore also be implemented as a module or related component in a set of logic instructions stored in a machine- or computer-readable storage medium such as RAM, ROM, PROM, firmware, flash memory, etc., in configurable logic such as, for example, PLAs, FPGAs, CPLDs, in fixed-functionality hardware logic using circuit technology such as, for example, ASIC, CMOS or TTL technology, or any combination thereof.

Illustrated block 28 provides for selecting a usage profile associated with the second energy pattern based on one or more of an activity (e.g., casual walking, power walking, jogging, running, cycling), a location (e.g., street address, Global Positioning System/GPS coordinates, etc.) or an interpersonal proximity (e.g., near family, friends, social network, etc.). Information from, for example, a user interface (UI), proximity sensor and/or social networking site/database, may be used to select the usage profile. Block 30 may select the first energy generation pattern from a plurality of training patterns based on the usage profile. In this regard, the authorized user may create multiple training patterns for various different scenarios (e.g., jogging in the neighborhood, walking at the mall, cycling on a trail) under which authentication might be requested.

Thus, if it is determined, for example, that the authentication session is being conducted while the individual to which the wearable device is coupled is jogging in a particular neighborhood (e.g., having a hilly topography), block 30 may select a training pattern that was also created while the authorized individual was jogging in that particular (or a similarly hilly) neighborhood. If, on the other hand, it is determined that, for example, that the authentication session is being conducted while the individual to which the wearable device is coupled is walking in a mall with a particular group of friends, block 30 might select a training pattern that was created while the authorized individual was walking in the same mall with the same group of friends. Of particular note is that the contextual information provided by the illustrated profile-based approach may reduce the likelihood of successful gait mimicking by unauthorized individuals.

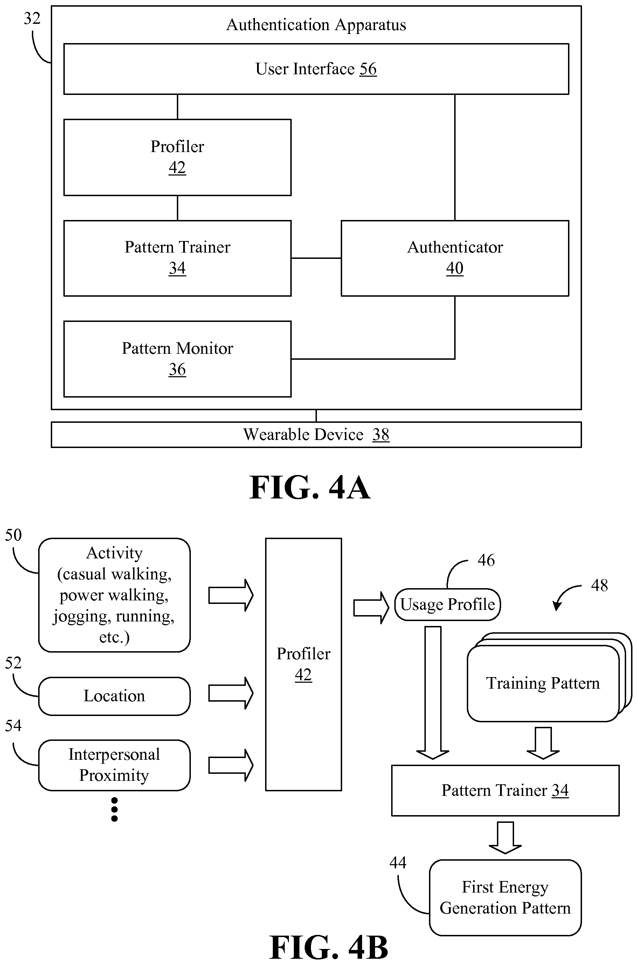

Turning now to FIG. 4A, a user-based system including an authentication apparatus 32 is shown, wherein the authentication apparatus 32 may be used in conjunction with a wearable device 38 such as, for example, the wearable device 12 (FIGs. 1 and 2), to authenticate users/individuals. Thus, the authentication apparatus 32 may generally implement one or more aspects of the method 18 (FIG. 3A) and/or the method 26 (FIG. 3B), already discussed. In the illustrated example, a pattern trainer 34 determines a first energy generation pattern associated with the wearable device 38 and a pattern monitor 36 determines a second energy generation pattern associated with the wearable device 38. More particularly, the pattern monitor 36 may monitor the wearable device 38 during an authentication session, wherein the authentication session may be initiated by a gesture input (e.g., screen tap, screen swipe, button push) or other suitable trigger. Additionally, an authenticator 40 may conduct a user authentication based at least in part on the first energy generation pattern and the second energy generation pattern. The authenticator 40 may compare the first energy generation pattern to the second energy generation pattern, wherein the energy generation patterns may be a function of weight, gait rhythm, step characteristics (e.g., level of pronation of the foot), and so forth. Accuracy may be further enhanced by combining the comparison results with other authentication techniques. For example, an energy profile that does not match the expected profile may trigger an authentication by some other method that may require the user's attention and/or may require additional use of power for another interface.

FIG. 4B demonstrates that a profiler 42 may select a usage profile 46 associated with the second energy generation pattern, wherein the pattern trainer 34 is to select the first energy generation pattern 44 from a plurality of training patterns 48 based on the usage profile 46. As already noted, the usage profile 46 may be selected based on activity information 50, location information 52, interpersonal proximity information 54, etc., associated with the authentication session/second energy generation pattern. The activity information 50 might include an indication of, for example, casual walking, power walking, jogging, running, cycling, and so forth. The activity information 50, location information 52 and/or interpersonal proximity information 54 may be obtained via a user interface 56 of the authentication apparatus 32 or other suitable source (e.g., GPS sensor, social networking site/database, etc.).

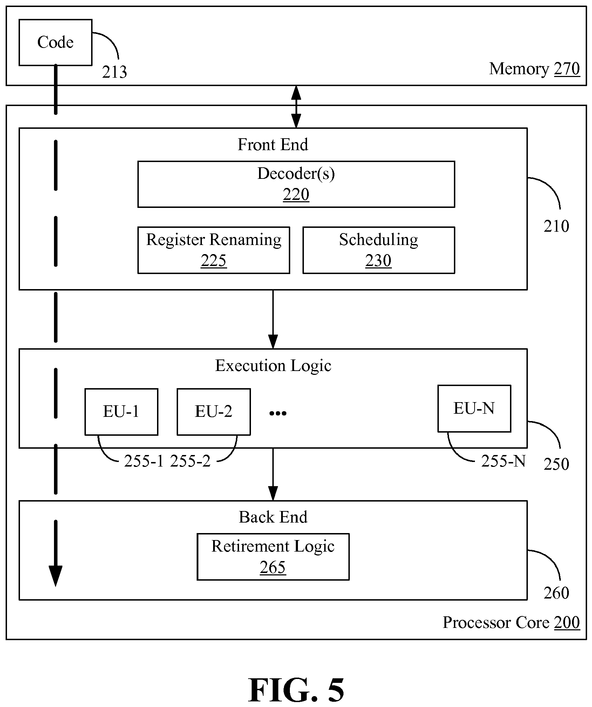

FIG. 5 illustrates a processor core 200 according to one embodiment. The processor core 200 may be the core for any type of processor, such as a micro-processor, an embedded processor, a digital signal processor (DSP), a network processor, or other device to execute code. Although only one processor core 200 is illustrated in FIG. 5, a processing element may alternatively include more than one of the processor core 200 illustrated in FIG. 5. The processor core 200 may be a single-threaded core or, for at least one embodiment, the processor core 200 may be multithreaded in that it may include more than one hardware thread context (or "logical processor") per core.

FIG. 5 also illustrates a memory 270 coupled to the processor core 200. The memory 270 may be any of a wide variety of memories (including various layers of memory hierarchy) as are known or otherwise available to those of skill in the art. The memory 270 may include one or more code 213 instruction(s) to be executed by the processor core 200, wherein the code 213 may implement the method 18 (FIG. 3A) and/or the method 26 (FIG. 3B), already discussed. In one example, the memory 270 is non-flash memory. The processor core 200 follows a program sequence of instructions indicated by the code 213. Each instruction may enter a front end portion 210 and be processed by one or more decoders 220. The decoder 220 may generate as its output a micro operation such as a fixed width micro operation in a predefined format, or may generate other instructions, microinstructions, or control signals which reflect the original code instruction. The illustrated front end portion 210 also includes register renaming logic 225 and scheduling logic 230, which generally allocate resources and queue the operation corresponding to the convert instruction for execution.

The processor core 200 is shown including execution logic 250 having a set of execution units 255-1 through 255-N. Some embodiments may include a number of execution units dedicated to specific functions or sets of functions. Other embodiments may include only one execution unit or one execution unit that can perform a particular function. The illustrated execution logic 250 performs the operations specified by code instructions.

After completion of execution of the operations specified by the code instructions, back end logic 260 retires the instructions of the code 213. In one embodiment, the processor core 200 allows out of order execution but requires in order retirement of instructions. Retirement logic 265 may take a variety of forms as known to those of skill in the art (e.g., re-order buffers or the like). In this manner, the processor core 200 is transformed during execution of the code 213, at least in terms of the output generated by the decoder, the hardware registers and tables utilized by the register renaming logic 225, and any registers (not shown) modified by the execution logic 250.

Although not illustrated in FIG. 5, a processing element may include other elements on chip with the processor core 200. For example, a processing element may include memory control logic along with the processor core 200. The processing element may include I/O control logic and/or may include I/O control logic integrated with memory control logic. The processing element may also include one or more caches.

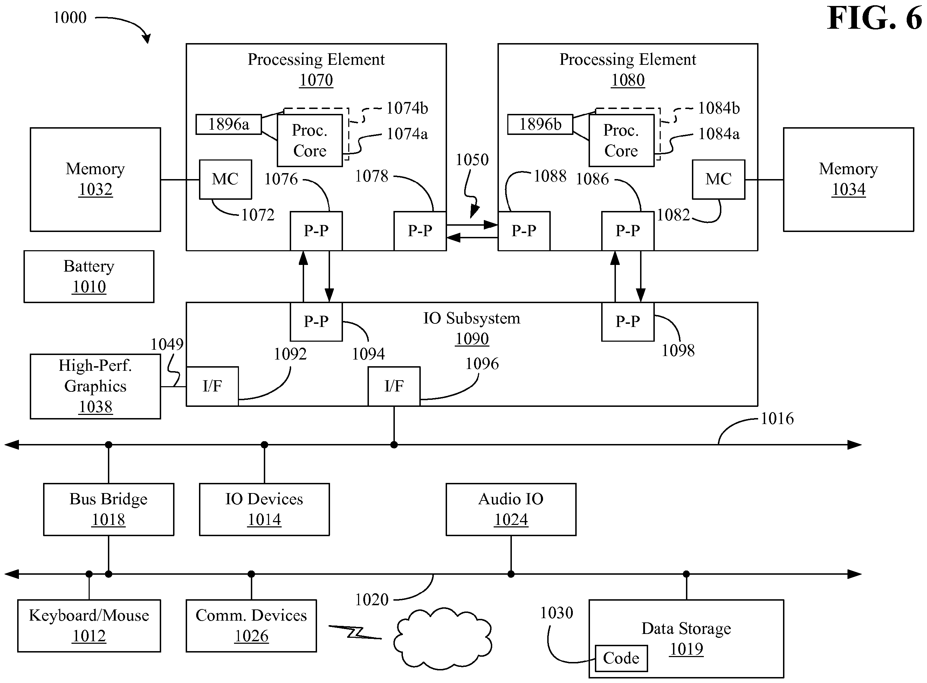

Referring now to FIG. 6, shown is a block diagram of a computing system 1000 embodiment in accordance with an embodiment. Shown in FIG. 6 is a multiprocessor system 1000 that includes a first processing element 1070 and a second processing element 1080. While two processing elements 1070 and 1080 are shown, it is to be understood that an embodiment of the system 1000 may also include only one such processing element.

The system 1000 is illustrated as a point-to-point interconnect system, wherein the first processing element 1070 and the second processing element 1080 are coupled via a point-to-point interconnect 1050. It should be understood that any or all of the interconnects illustrated in FIG. 6 may be implemented as a multi-drop bus rather than point-to-point interconnect.

As shown in FIG. 6, each of processing elements 1070 and 1080 may be multicore processors, including first and second processor cores (i.e., processor cores 1074a and 1074b and processor cores 1084a and 1084b). Such cores 1074a, 1074b, 1084a, 1084b may be configured to execute instruction code in a manner similar to that discussed above in connection with FIG. 5.

Each processing element 1070, 1080 may include at least one shared cache 1896a, 1896b. The shared cache 1896a, 1896b may store data (e.g., instructions) that are utilized by one or more components of the processor, such as the cores 1074a, 1074b and 1084a, 1084b, respectively. For example, the shared cache 1896a, 1896b may locally cache data stored in a memory 1032, 1034 for faster access by components of the processor. In one or more embodiments, the shared cache 1896a, 1896b may include one or more mid-level caches, such as level 2 (L2), level 3 (L3), level 4 (L4), or other levels of cache, a last level cache (LLC), and/or combinations thereof.

While shown with only two processing elements 1070, 1080, it is to be understood that the scope of the embodiments are not so limited. In other embodiments, one or more additional processing elements may be present in a given processor. Alternatively, one or more of processing elements 1070, 1080 may be an element other than a processor, such as an accelerator or a field programmable gate array. For example, additional processing element(s) may include additional processors(s) that are the same as a first processor 1070, additional processor(s) that are heterogeneous or asymmetric to processor a first processor 1070, accelerators (such as, e.g., graphics accelerators or digital signal processing (DSP) units), field programmable gate arrays, or any other processing element. There can be a variety of differences between the processing elements 1070, 1080 in terms of a spectrum of metrics of merit including architectural, micro architectural, thermal, power consumption characteristics, and the like. These differences may effectively manifest themselves as asymmetry and heterogeneity amongst the processing elements 1070, 1080. For at least one embodiment, the various processing elements 1070, 1080 may reside in the same die package.

The first processing element 1070 may further include memory controller logic (MC) 1072 and point-to-point (P-P) interfaces 1076 and 1078. Similarly, the second processing element 1080 may include a MC 1082 and P-P interfaces 1086 and 1088. As shown in FIG. 6, MC's 1072 and 1082 couple the processors to respective memories, namely a memory 1032 and a memory 1034, which may be portions of main memory locally attached to the respective processors. While the MC 1072 and 1082 is illustrated as integrated into the processing elements 1070, 1080, for alternative embodiments the MC logic may be discrete logic outside the processing elements 1070, 1080 rather than integrated therein.

The first processing element 1070 and the second processing element 1080 may be coupled to an I/O subsystem 1090 via P-P interconnects 1076 1086, respectively. As shown in FIG. 6, the I/O subsystem 1090 includes P-P interfaces 1094 and 1098. Furthermore, I/O subsystem 1090 includes an interface 1092 to couple I/O subsystem 1090 with a high performance graphics engine 1038. In one embodiment, bus 1049 may be used to couple the graphics engine 1038 to the I/O subsystem 1090. Alternately, a point-to-point interconnect may couple these components.

In turn, I/O subsystem 1090 may be coupled to a first bus 1016 via an interface 1096. In one embodiment, the first bus 1016 may be a Peripheral Component Interconnect (PCI) bus, or a bus such as a PCI Express bus or another third generation I/O interconnect bus, although the scope of the embodiments are not so limited.

As shown in FIG. 6, various I/O devices 1014 (e.g., speakers, cameras, sensors) may be coupled to the first bus 1016, along with a bus bridge 1018 which may couple the first bus 1016 to a second bus 1020. In one embodiment, the second bus 1020 may be a low pin count (LPC) bus. Various devices may be coupled to the second bus 1020 including, for example, a keyboard/mouse 1012, communication device(s) 1026, and a data storage unit 1019 such as a disk drive or other mass storage device which may include code 1030, in one embodiment. The illustrated code 1030 may implement the method 18 (FIG. 3A) and/or the method 26 (FIG. 3B), already discussed, and may be similar to the code 213 (FIG. 5), already discussed. Further, an audio I/O 1024 may be coupled to second bus 1020 and a battery 1010 may supply power to the computing system 1000.

Note that other embodiments are contemplated. For example, instead of the point-to-point architecture of FIG. 6, a system may implement a multi-drop bus or another such communication topology. Also, the elements of FIG. 6 may alternatively be partitioned using more or fewer integrated chips than shown in FIG. 6.

### Additional Notes and Examples:

Example 1 may include a user-based system comprising a wearable device including an energy generation component, a pattern trainer to determine a first energy generation pattern associated with the wearable device, a pattern monitor to determine a second energy generation pattern associated with the wearable device and an authenticator to conduct a user authentication based at least in part on the first energy generation pattern and the second energy generation pattern.
Example 2 may include the system of Example 1, further including a profiler to select a usage profile associated with the second energy generation pattern, wherein the pattern trainer is to select the first energy generation pattern from a plurality of training patterns based on the usage profile.
Example 3 may include the system of Example 2, wherein the usage profile is to be selected based on one or more of an activity, a location or an interpersonal proximity.
Example 4 may include the system of Example 3, wherein the activity is to include one or more of casual walking, power walking, jogging, running or cycling.
Example 5 may include the system of Example 1, wherein the pattern monitor is to monitor the wearable device during an authentication session initiated by a user input to determine the second energy generation pattern.
Example 6 may include the system of any one of Examples 1 to 5, wherein the authenticator is to compare the first energy generation pattern to the second energy generation pattern with respect to one or more of a weight, a gait rhythm or a step characteristic.
Example 7 may include the system of any one of Examples 1 to 5, wherein the wearable device has a footwear form factor and the energy generation component includes one or more piezoelectric sensors.
Example 8 may include an authentication apparatus comprising a pattern trainer to determine a first energy generation pattern associated with a wearable device, a pattern monitor to determine a second energy generation pattern associated with the wearable device and an authenticator to conduct a user authentication based at least in part on the first energy generation pattern and the second energy generation pattern.
Example 9 may include the apparatus of Example 8, further including a profiler to select a usage profile associated with the second energy generation pattern, wherein the pattern trainer is to select the first energy generation pattern from a plurality of training patterns based on the usage profile.
Example 10 may include the apparatus of Example 9, wherein the usage profile is to be selected based on one or more of an activity, a location or an interpersonal proximity.
Example 11 may include the apparatus of Example 10, wherein the activity is to include one or more of casual walking, power walking, jogging, running or cycling.
Example 12 may include the apparatus of Example 8, wherein the pattern monitor is to monitor the wearable device during an authentication session initiated by a user input to determine the second energy generation pattern.
Example 13 may include the apparatus of any one of Examples 8 to 12, wherein the authenticator is to compare the first energy generation pattern to the second energy generation pattern with respect to one or more of a weight, a gait rhythm or a step characteristic.
Example 14 may include a method of authenticating users comprising determining a first energy generation pattern associated with a wearable device, determining a second energy generation pattern associated with the wearable device and conducting a user authentication based at least in part on the first energy generation pattern and the second energy generation pattern.
Example 15 may include the method of Example 14, further including selecting a usage profile associated with the second energy generation pattern, wherein determining the first energy generation pattern includes selecting the first energy generation pattern from a plurality of training patterns based on the usage profile.
Example 16 may include the method of Example 15, wherein the usage profile is selected based on one or more of an activity, a location or an interpersonal proximity.
Example 17 may include the method of Example 16, wherein the activity includes one or more of casual walking, power walking, jogging, running or cycling.
Example 18 may include the method of Example 14, wherein determining the second energy generation pattern includes monitoring the wearable device during an authentication session initiated by a user input.
Example 19 may include the method of any one of Examples 14 to 18, wherein conducting the user authentication includes comparing the first energy generation pattern to the second energy generation pattern with respect to one or more of a weight, a gait rhythm or a step characteristic.
Example 20 may include at least one computer readable storage medium comprising a set of instructions which, when executed, cause a computing device to determine a first energy generation pattern associated with a wearable device, determine a second energy generation pattern associated with the wearable device and conduct a user authentication based at least in part on the first energy generation pattern and the second energy generation pattern.
Example 21 may include the at least one computer readable storage medium of Example 20, wherein the instructions, when executed, cause a computing device to select a usage profile associated with the second energy generation pattern; and select the first energy generation pattern from a plurality of training patterns based on the usage profile.
Example 22 may include the at least one computer readable storage medium of Example 21, wherein the usage profile is to be selected based on one or more of an activity, a location or an interpersonal proximity.
Example 23 may include the at least one computer readable storage medium of Example 22, wherein the activity is to include one or more of casual walking, power walking, jogging, running or cycling.
Example 24 may include the at least one computer readable storage medium of Example 20, wherein the instructions, when executed, cause a computing device to monitor the wearable device during an authentication session initiated by a user input to determine the second energy generation pattern.
Example 25 may include the at least one computer readable storage medium of any one of Examples 20 to 24, wherein the instructions, when executed, cause a computing device to compare the first energy generation pattern to the second energy generation pattern with respect to one or more of a weight, a gait rhythm or a step characteristic.
Example 26 may include an authentication apparatus comprising means for determining a first energy generation pattern associated with a wearable device; means for determining a second energy generation pattern associated with the wearable device; and means for conducting a user authentication based at least in part on the first energy generation pattern and the second energy generation pattern.
Example 27 may include the apparatus of Example 26, further including means for selecting a usage profile associated with the second energy generation pattern, wherein the means for determining the first energy generation pattern includes means for selecting the first energy generation pattern from a plurality of training patterns based on the usage profile.
Example 28 may include the apparatus of Example 27, wherein the usage profile is to be selected based on one or more of an activity, a location or an interpersonal proximity.
Example 29 may include the apparatus of Example 28, wherein the activity is to include one or more of casual walking, power walking, jogging, running or cycling.
Example 30 may include the apparatus of Example 26, wherein the means for determining the second energy generation pattern includes means for monitoring the wearable device during an authentication session initiated by a user input.
Example 31 may include the apparatus of any one of Examples 26 to 30, wherein the means for conducting the user authentication includes means for comparing the first energy generation pattern to the second energy generation pattern with respect to one or more of a weight, a gait rhythm or a step characteristic.

Thus, techniques described herein may collect energy harvesting data to create energy generation profiles of a wearer in different contexts. Additionally, the appropriate energy generation profile may be used to authenticate the user. Factors such as weight, gait rhythm and step characteristics may influence the profile. Moreover, authentication coordination may involve obtaining input from power profile modules and/or multiple sensors, depending on the circumstances, and determining which factors to use in the authentication.

Embodiments are applicable for use with all types of semiconductor integrated circuit ("IC") chips. Examples of these IC chips include but are not limited to processors, controllers, chipset components, programmable logic arrays (PLAs), memory chips, network chips, systems on chip (SoCs), SSD/NAND controller ASICs, and the like. In addition, in some of the drawings, signal conductor lines are represented with lines. Some may be different, to indicate more constituent signal paths, have a number label, to indicate a number of constituent signal paths, and/or have arrows at one or more ends, to indicate primary information flow direction. This, however, should not be construed in a limiting manner. Rather, such added detail may be used in connection with one or more exemplary embodiments to facilitate easier understanding of a circuit. Any represented signal lines, whether or not having additional information, may actually comprise one or more signals that may travel in multiple directions and may be implemented with any suitable type of signal scheme, e.g., digital or analog lines implemented with differential pairs, optical fiber lines, and/or single-ended lines.

Example sizes/models/values/ranges may have been given, although embodiments are not limited to the same. As manufacturing techniques (e.g., photolithography) mature over time, it is expected that devices of smaller size could be manufactured. In addition, well known power/ground connections to IC chips and other components may or may not be shown within the figures, for simplicity of illustration and discussion, and so as not to obscure certain aspects of the embodiments. Further, arrangements may be shown in block diagram form in order to avoid obscuring embodiments, and also in view of the fact that specifics with respect to implementation of such block diagram arrangements are highly dependent upon the computing system within which the embodiment is to be implemented, i.e., such specifics should be well within purview of one skilled in the art. Where specific details (e.g., circuits) are set forth in order to describe example embodiments, it should be apparent to one skilled in the art that embodiments can be practiced without, or with variation of, these specific details. The description is thus to be regarded as illustrative instead of limiting.

The term "coupled" may be used herein to refer to any type of relationship, direct or indirect, between the components in question, and may apply to electrical, mechanical, fluid, optical, electromagnetic, electromechanical or other connections. In addition, the terms "first", "second", etc. may be used herein only to facilitate discussion, and carry no particular temporal or chronological significance unless otherwise indicated.

As used in this application and in the claims, a list of items joined by the term "one or more of' may mean any combination of the listed terms. For example, the phrases "one or more of A, B or C" may mean A; B; C; A and B; A and C; B and C; or A, B and C.

Those skilled in the art will appreciate from the foregoing description that the broad techniques of the embodiments can be implemented in a variety of forms. Therefore, while the embodiments have been described in connection with particular examples thereof, the true scope of the embodiments should not be so limited since other modifications will become apparent to the skilled practitioner upon a study of the drawings, specification, and following claims.

## Claims

1. A user-based system comprising:
a wearable device (12) including an energy generation component (14);
a pattern trainer (34) to determine, based on an activity of a user of the wearable device (12), a first energy generation pattern generated by the energy generation component and associated with the wearable device (12);
a pattern monitor (36) to determine, based on another activity of the user of the wearable device (12), a second energy generation pattern generated by the energy generation component and associated with the wearable device (12); and
an authenticator (40) to conduct a user authentication, of the user of the wearable device, based at least in part on the first energy generation pattern and the second energy generation pattern.

2. The system of claim 1, further including a profiler (42) to select a usage profile associated with the second energy generation pattern, wherein the pattern trainer (34) is to select the first energy generation pattern from a plurality of training patterns based on the usage profile.

3. The system of claim 2, wherein the usage profile is to be selected based on one or more of an activity, a location or an interpersonal proximity.

4. The system of claim 3, wherein the activity is to include one or more of casual walking, power walking, jogging, running or cycling.

5. The system of claim 1, wherein the pattern monitor (36) is to monitor the wearable device (12) during an authentication session initiated by a user input to determine the second energy generation pattern.

6. The system of any one of claims 1 to 5, wherein the authenticator (40) is to compare the first energy generation pattern to the second energy generation pattern with respect to one or more of a weight, a gait rhythm or a step characteristic.

7. The system of any one of claims 1 to 5, wherein the wearable device (12) has a footwear form factor and the energy generation component includes one or more piezoelectric sensors (14).

8. A method of authenticating users, comprising:
determining, based on an activity of a user of the wearable device (12), a first energy generation pattern generated by an energy generation component (14) and associated with a wearable device (12);
determining, based on another activity of the user of the wearable device (12), a second energy generation pattern generated by the energy generation component (14) and associated with the wearable device (12); and
conducting a user authentication, of the user of the wearable device, based at least in part on the first energy generation pattern and the second energy generation pattern.

9. The method of claim 8, further including selecting a usage profile associated with the second energy generation pattern, wherein determining the first energy generation pattern includes selecting the first energy generation pattern from a plurality of training patterns based on the usage profile.

10. The method of claim 9, wherein the usage profile is selected based on one or more of an activity, a location or an interpersonal proximity.

11. The method of claim 10, wherein the activity includes one or more of casual walking, power walking, jogging, running or cycling.

12. The method of claim 8, wherein determining the second energy generation pattern includes monitoring the wearable device (12) during an authentication session initiated by a user input.

13. The method of any one of claims 8 to 12, wherein conducting the user authentication includes comparing the first energy generation pattern to the second energy generation pattern with respect to one or more of a weight, a gait rhythm or a step characteristic.

14. At least one computer readable storage medium comprising a set of instructions which, when executed by a computing device, cause the computing device to:
determine, based on an activity of a user of the wearable device (12), a first energy generation pattern generated by an energy generation component and associated with a wearable device;
determine, based on another activity of the user of the wearable device (12), a second energy generation pattern generated by the energy generation component and associated with the wearable device (12); and
conduct a user authentication, of the user of the wearable device, based at least in part on the first energy generation pattern and the second energy generation pattern.

15. The at least one computer readable storage medium of claim 14, wherein the instructions, when executed, cause a computing device to:
select a usage profile associated with the second energy generation pattern; and
select the first energy generation pattern from a plurality of training patterns based on the usage profile.

## Patentansprüche

1. Benutzerbasiertes System, umfassend:
eine tragbare Einrichtung (12) einschließlich einer Energieerzeugungskomponente (14);
einen Mustertrainer (34) zum Bestimmen, basierend auf einer Aktivität eines Benutzers der tragbaren Einrichtung (12), eines ersten Energieerzeugungsmusters, das durch die Energieerzeugungskomponente erzeugt wird und mit der tragbaren Einrichtung (12) assoziiert ist;
eine Musterüberwachungsvorrichtung (36) zum Bestimmen, basierend auf einer anderen Aktivität des Benutzers der tragbaren Einrichtung (12), eines zweiten Energieerzeugungsmusters, das durch die Energieerzeugungskomponente erzeugt wird und mit der tragbaren Einrichtung (12) assoziiert ist; und
einen Authentifizierer (40) zum Durchführen einer Benutzerauthentifizierung des Benutzers der tragbaren Einrichtung zumindest teilweise basierend auf dem ersten Energieerzeugungsmuster und dem zweiten Energieerzeugungsmuster.

2. System nach Anspruch 1, das ferner eine Profileinheit (42) zum Auswählen eines Nutzungsprofils, das mit dem zweiten Energieerzeugungsmuster assoziiert ist, beinhaltet, wobei der Mustertrainer (34) das erste Energieerzeugungsmuster basierend auf dem Nutzungsprofil aus mehreren Trainingsmustern auswählen soll.

3. System nach Anspruch 2, wobei das Nutzungsprofil basierend auf einer Aktivität und/oder einem Standort und/oder einer zwischenmenschlichen Nähe ausgewählt werden soll.

4. System nach Anspruch 3, wobei die Aktivität gemächliches Laufen und/oder Power-Walking und/oder Jogging und/oder Rennen und/oder Fahrradfahren beinhalten soll.

5. System nach Anspruch 1, wobei die Musterüberwachungsvorrichtung (36) die tragbare Einrichtung (12) während einer Authentifizierungssitzung, die durch eine Benutzereingabe initiiert wird, überwachen soll, um das zweite Energieerzeugungsmuster zu bestimmen.

6. System nach einem der Ansprüche 1 bis 5, wobei der Authentifizierer (40) das erste Energieerzeugungsmuster mit dem zweiten Energieerzeugungsmuster bezüglich eines Gewichts und/oder eines Gangrhythmus und/oder einer Schrittcharakteristik vergleichen soll.

7. System nach einem der Ansprüche 1 bis 5, wobei die tragbare Einrichtung (12) einen Schuhwerkformfaktor aufweist und die Energieerzeugungskomponente einen oder mehrere piezoelektrische Sensoren (14) beinhaltet.

8. Verfahren zur Authentifizierung von Benutzern, umfassend:
Bestimmen, basierend auf einer Aktivität eines Benutzers der tragbaren Einrichtung (12), eines ersten Energieerzeugungsmusters, das durch eine Energieerzeugungskomponente (14) erzeugt wird und mit einer tragbaren Einrichtung (12) assoziiert ist;
Bestimmen, basierend auf einer anderen Aktivität des Benutzers der tragbaren Einrichtung (12), eines zweiten Energieerzeugungsmusters, das durch die Energieerzeugungskomponente (14) erzeugt wird und mit der tragbaren Einrichtung (12) assoziiert ist; und
Durchführen einer Benutzerauthentifizierung des Benutzers der tragbaren Einrichtung zumindest teilweise basierend auf dem ersten Energieerzeugungsmuster und dem zweiten Energieerzeugungsmuster.

9. Verfahren nach Anspruch 8, das ferner Auswählen eines Nutzungsprofils, das mit dem zweiten Energieerzeugungsmuster assoziiert ist, beinhaltet, wobei das Bestimmen des ersten Energieerzeugungsmusters ein Auswählen des ersten Energieerzeugungsmusters aus mehreren Trainingsmustern basierend auf dem Nutzungsprofil beinhaltet.

10. Verfahren nach Anspruch 9, wobei das Nutzungsprofil basierend auf einer Aktivität und/oder einem Standort und/oder einer zwischenmenschlichen Nähe ausgewählt wird.

11. Verfahren nach Anspruch 10, wobei die Aktivität gemächliches Laufen und/oder Power-Walking und/oder Jogging und/oder Rennen und/oder Fahrradfahren beinhaltet.

12. Verfahren nach Anspruch 8, wobei das Bestimmen des zweiten Energieerzeugungsmusters ein Überwachen der tragbaren Einrichtung (12) während einer Authentifizierungssitzung, die durch eine Benutzereingabe initiiert wird, beinhaltet.

13. Verfahren nach einem der Ansprüche 8 bis 12, wobei das Durchführen der Benutzerauthentifizierung ein Vergleichen des ersten Energieerzeugungsmusters mit dem zweiten Energieerzeugungsmuster bezüglich eines Gewichts und/oder eines Gangrhythmus und/oder einer Schrittcharakteristik beinhaltet.

14. Computerlesbares Speichermedium bzw. computerlesbare Speichermedien, die einen Satz von Anweisungen umfassen, die bei Ausführung durch eine Recheneinrichtung die Recheneinrichtung zu Folgendem veranlassen:
Bestimmen, basierend auf einer Aktivität eines Benutzers der tragbaren Einrichtung (12), eines ersten Energieerzeugungsmusters, das durch eine Energieerzeugungskomponente erzeugt wird und mit einer tragbaren Einrichtung assoziiert ist;
Bestimmen, basierend auf einer anderen Aktivität des Benutzers der tragbaren Einrichtung (12), eines zweiten Energieerzeugungsmusters, das durch die Energieerzeugungskomponente erzeugt wird und mit der tragbaren Einrichtung (12) assoziiert ist; und
Durchführen einer Benutzerauthentifizierung des Benutzers der tragbaren Einrichtung zumindest teilweise basierend auf dem ersten Energieerzeugungsmuster und dem zweiten Energieerzeugungsmuster.

15. Computerlesbares Speichermedium bzw. computerlesbare Speichermedien nach Anspruch 14, wobei die Anweisungen bei Ausführung eine Recheneinrichtung zu Folgendem veranlassen:
Auswählen eines Nutzungsprofil, das mit dem zweiten Energieerzeugungsmuster assoziiert ist; und
Auswählen des ersten Energieerzeugungsmusters aus mehreren Trainingsmustern basierend auf dem Nutzungsprofil.

## Revendications

1. Système basé sur l'utilisateur comprenant :
un dispositif portable (12) comprenant un composant de génération d'énergie (14) ;
un formateur de modèle (34) pour déterminer, sur la base d'une activité d'un utilisateur du dispositif portable (12), un premier modèle de génération d'énergie généré par le composant de génération d'énergie et associé au dispositif portable (12) ;
un moniteur de modèle (36) pour déterminer, sur la base d'une autre activité de l'utilisateur du dispositif portable (12), un second modèle de génération d'énergie généré par le composant de génération d'énergie et associé au dispositif portable (12) ; et
un authentificateur (40) pour effectuer l'authentification en tant qu'utilisateur de l'utilisateur du dispositif portable, sur la base au moins en partie du premier modèle de génération d'énergie et du second modèle de génération d'énergie.

2. Système selon la revendication 1, comprenant en outre un générateur de profil (42) pour sélectionner un profil d'utilisation associé au second modèle de génération d'énergie, dans lequel le formateur de modèle (34) doit sélectionner le premier modèle de génération d'énergie parmi une pluralité de modèles de formation sur la base du profil d'utilisation.

3. Système selon la revendication 2, dans lequel le profil d'utilisation doit être sélectionné sur la base d'un ou de plusieurs parmi une activité, un lieu ou une proximité interpersonnelle.

4. Système selon la revendication 3, dans lequel l'activité doit comprendre une ou plusieurs activités parmi la marche ordinaire, la marche rapide, le jogging, la course ou le cyclisme.

5. Système selon la revendication 1, dans lequel le moniteur de modèle (36) doit surveiller le dispositif portable (12) pendant une session d'authentification lancée par une entrée d'utilisateur pour déterminer le second modèle de génération d'énergie.

6. Système selon l'une quelconque des revendications 1 à 5, dans lequel l'authentificateur (40) doit comparer le premier modèle de génération d'énergie au second modèle de génération d'énergie par rapport à une ou plusieurs caractéristiques parmi une caractéristique de poids, de rythme de marche ou de pas.

7. Système selon l'une quelconque des revendications 1 à 5, dans lequel le dispositif portable (12) a un facteur de forme de chaussure et le composant de génération d'énergie comprend un ou plusieurs capteurs piézoélectriques (14).

8. Procédé d'authentification des utilisateurs, comprenant les étapes consistant à :
déterminer, sur la base d'une activité d'un utilisateur du dispositif portable (12), un premier modèle de génération d'énergie généré par un composant de génération d'énergie (14) et associé à un dispositif portable (12) ;
déterminer, sur la base d'une autre activité de l'utilisateur du dispositif portable (12), un second modèle de génération d'énergie généré par le composant de génération d'énergie (14) et associé au dispositif portable (12) ; et
procéder à une authentification en tant qu'utilisateur de l'utilisateur du dispositif portable, sur la base au moins en partie du premier modèle de génération d'énergie et du second modèle de génération d'énergie.

9. Procédé selon la revendication 8, comprenant en outre la sélection d'un profil d'utilisation associé au second modèle de génération d'énergie, dans lequel la détermination du premier modèle de génération d'énergie comprend la sélection du premier modèle de production d'énergie parmi une pluralité de modèles de formation sur la base du profil d'utilisation.

10. Procédé selon la revendication 9, dans lequel le profil d'utilisation est sélectionné sur la base d'un ou de plusieurs parmi une activité, un lieu ou une proximité interpersonnelle.

11. Procédé selon la revendication 10, dans lequel l'activité doit comprendre une ou plusieurs activités parmi la marche ordinaire, la marche rapide, le jogging, la course ou le cyclisme.

12. Procédé selon la revendication 8, dans lequel la détermination du second modèle de génération d'énergie comprend la surveillance du dispositif portable (12) au cours d'une session d'authentification lancée par une entrée de l'utilisateur.

13. Procédé selon l'une quelconque des revendications 8 à 12, dans lequel la réalisation de l'authentification de l'utilisateur comprend la comparaison du premier modèle de génération d'énergie au second modèle de génération d'énergie par rapport à une ou plusieurs caractéristiques parmi une caractéristique de poids, de rythme de marche ou de pas.

14. Au moins un support de stockage lisible par ordinateur comprenant un ensemble d'instructions qui, lorsqu'il est exécuté par un dispositif informatique, amène le dispositif informatique à :
déterminer, sur la base d'une activité d'un utilisateur du dispositif portable (12), un premier modèle de génération d'énergie généré par un composant de génération d'énergie et associé à un dispositif portable ;
déterminer, sur la base d'une autre activité de l'utilisateur du dispositif portable (12), un second modèle de génération d'énergie généré par le composant de génération d'énergie et associé au dispositif portable ; et
effectuer une authentification en tant qu'utilisateur de l'utilisateur du dispositif portable, sur la base au moins en partie du premier modèle de génération d'énergie et du second modèle de génération d'énergie.

15. Au moins un support de stockage lisible par ordinateur selon la revendication 14, dans lequel les instructions, lorsqu'elles sont exécutées, amènent un dispositif informatique à :
sélectionner un profil d'utilisation associé au second modèle de génération d'énergie ; et
sélectionner le premier modèle de génération d'énergie parmi une pluralité de modèles de formation sur la base du profil d'utilisation.
